# EUROPEAN PATENT APPLICATION

(11) **EP 1 946 746 A1**
(43) Date of publication of application: **23.07.2008**
(21) Application number: 07000945.1
(22) Date of filing: 17.01.2007
(51) Int. Cl.: A61K 9/00, A61K 9/08, A61K 9/10, A61K 47/12, A61K 47/14

(54) **Particulate aqueous system for the preparation of a formulation for the treatment of adipose diseases**

(71) Applicant: Merz Pharma GmbH & Co. KGaA, 60318 Frankfurt Main (DE)
(72) Inventor: Mentrup, Edgard, Dr., 60437 Frankfurt am Main (DE); Pooth, Rainer, Dr., 68512 Bad Soden/Taununs (DE)
(74) Representative: Hüttermann, Aloys

(57) **Abstract**

Aqueous particulate system comprising at least one water dispersible biopolymer, at least one surfactant, and water for the preparation of a formulation for the treatment of adipose diseases and/or conditions with improved bioavailability and good retardation of the release of active substances.

## Description

The present invention relates to the use of an aqueous particulate system comprising at least one water dispersible biopolymer, at least one surfactant, and water for the preparation of a formulation for the treatment of adipose diseases and/or conditions.

In general, aqueous systems comprising of water dispersible biopolymers, surfactants, and water are well known in the field of chemical formulations to act as carrier systems in drug delivery (Martin Malmsten, Surfactants and Polymers in Drug Delivery, publisher Marcel Dekker, 2002). They can allow control of the drug release rate, enhance effective drug solubility, minimize drug degradation, and contribute to reduced toxicity. In all, they contribute to therapeutic efficiency of the active ingredient itself but they are not intended to have any pharmaceutically active effects. That would contravene their target function as a drug carrier system.

On the other hand, aqueous systems of phospholipids and bile acid or its derivatives are well known for the preparation of cosmetic and pharmaceutical formulations.
EP 0 615 746 A1 describes such formulations that can carry a pharmaceutically active substance or that can be used without an active drug. In the latter case, it is described that such liposomes can be used for the treatment of atherosclerosis, elevated blood lipids, and hepatopathy of any kind.
The described systems show a distinct liposome structure, i.e. a double membrane of lipids that encapsulates an aqueous phase.

In recent literature it is further described that such liposome systems can reduce fatty tissue when locally injected subcutaneously (Patricia Guedes Rittes, The use of phosphatidylcholine for correction of lower lid bulging due to prominent fat pads, Dermatol Surg 2001, 27, 391-392).

Further, a special liposome system for the prophylaxis and treatment of fatty embolism is known that comprises phospholipids, bile acid, DL-alpha-Tocopherole, ethanol and water (Lipostabil ® N i.V.).

However, the known aqueous liposome systems of phospholipids and bile acid or its derivatives for the treatment of reducing fatty tissue have the distinct disadvantage that their distribution inside the tissue is poor and thus the effect is fairly locally constricted to the immediate point of injection. Accordingly, up to date it is necessary for the treatment of a wider area of tissue to apply a high number of injections close to each other. Moreover, the release of the active substances is more or less immediate in the known systems which makes a higher number of repetitions in the treatment necessary. As mentioned earlier, the treatment is administered subcutaneously and multiple repetitions are therefore most inconvenient.

Therefore, it is an object of the present invention to provide a formulation for the treatment of adipose diseases and/or conditions that shows a good biocompatibility and an enhanced bioavailability together with a slow release rate such that wider areas of fatty tissue can be affected and the number of repetitive treatments can be reduced.

Surprisingly, it was found that the aqueous particulate system comprising of at least one water dispersible biopolymer, at least one surfactant, and water for the preparation of a formulation for the treatment of adipose diseases and/or conditions meets the object of the present invention.

The system of the present invention shows a better bioavailability and a better distribution in the fatty tissue. Thus, it allows for fewer injections when a wider area of tissue is to be treated and in general, a better effect of lipolysis. Furthermore, the inventive formulation shows a slow release rate of the active substances which also contributes to the fact that the number of injections can be reduced.
It is thought that the advantageous effects are derived due to the special interaction of the dispersed biopolymer with the surfactant, wherein the surfactant is believed to function as active ingredient. However, the transport mechanisms involved are not well understood so that scientifically sound evidence for the mechanism is yet to be found.

Under the term water dispersible biopolymer there are in particular homogeneous stable dispersions of particulate biopolymers with good wettability and excellent hydration ability in aqueous systems understood. The maximum particle size of the dispersed biopolymer is preferably between 5 µm and 500 µm, more preferably between 50 µm and 200 µm.

Most preferably, the water dispersible biopolymer is selected from the group comprising chitosan, alginates, 3,3-N-(aminoalkyl) chitosan, poly(D,L-lactic-)co-glycoside, lactic acid, glycolic acid, albumin, poly adipic acid anhydride, gellan gum, poly-L-lysine, and polypeptides.

Preferably, the surfactant of the present invention can be selected from the group comprising 3-sn-phosphatidylcholine, soy (phospholipone 90), reduced soy (phospholipon 90H), 3-(3sn)-phosphatidylgycerol soy (phospholipon G), dimyristoylphosphatidylglycerole, lyso-phophatidylcholine, dipalmitoylphosphatidylglycerole, and/or their physiologically acceptable salts, desoxycholic acid, cholic acid, lithocholic acid, chendodesoxycholic acid, hyodesoxycholic acid, trihydroxycoprostanic acid, ursodesoxycholic acid, taurocholic acid, or glycocholic acid and/or their physiologically acceptable salts, as well as ethers of ethoxylated alcohols and alkyl-alcohols (C6-C16), alkyl-ester with C8-C20 with ethoxylated alcohols, ester of saturated and unsatured acids with C8-C20 with sugars, alkylethersulfates like polyether of caster oil and ethylene oxide (cremephor EL), polyoxyethylene fatty alcohol ether, polysorbic monoester, poloxamer, poloxamine.

More preferably, the surfactant has an HLB value of between 5 and 15.

In another preferred embodiment of the present invention a lipophilic substance is added to the inventive system and can be selected from the group comprising natural oils (e.g. soy bean oil), ester of middle-chain alkylacids with glycols, octyl-dodecanol, silicon oils, paraffins, fatty acids and/or their esters, riboflavine, and/or L-camitine.

Due to the further addition of a lipophilic substance an even better distribution of the active substances can take place. Thus, wider areas of tissue can be affected by a single injection.

In a preferred embodiment of the present invention the system further comprises a co-surfactant.
In general, the co-surfactant is less lipophilic than the surfactant with an HLB value of 9 to 17.
Like that, a further stabilisation of the inventive aqueous particulate system can be achieved resulting in a longer shelf-life.

The co-surfactant can in particular be chosen from the group of 3-sn-phosphatidylcholine, soy (phospholipone 90), reduced soy (phospholipon 90H), 3-(3sn)-phosphatidylgycerol soy (phospholipon G), dimyristoylphosphatidylglycerole, lyso-phophatidylcholine, dipalmitoylphosphatidylglyCerole, and/or their physiologically acceptable salts, desoxycholic acid, cholic acid, lithocholic acid, chendodesoxycholic acid, hyodesoxycholic acid, trihydroxycoprostanic acid, ursodesoxycholic acid, taurocholic acid, or glycocholic acid and/or their physiologically acceptable salts, as well as ethers of ethoxylated alcohols and alkyl-alcohols (C6-C16), alkyl-ester with C8-C20 with ethoxylated alcohols, ester of saturated and unsatured acids with C8-C20 with sugars, alkylethersulfates like polyether of caster oil and ethylene oxide (cremephor EL), polyoxyethylene fatty alcohol ether, polysorbic monoester, poloxamer, poloxamine.

According to another preferred embodiment of the present invention the system additionally comprises an alcohol.
Particularly preferred alcohols are C2-C8 alcohols, and in particular ethanol, propylene glycol, and glycerine,

The mass ratio of the water dispersible biopolymer to the surfactant is preferably between 10: 1 and 1 : 10 weight %, more preferably from 1 : 0.2 and 1 : 1.5 weight %.
The concentration of the surfactant in the system is preferably between 0.5 and 50 weight %, in particular between 5 and 25 weight %.

The pH value of the system according to the present invention is neutral and ranges preferably between 5.0 and 9.0, more preferably between 6.0 and 8.0.

Under the term adipose disease and/or condition in particular the following diseases are understood:

Lipomae are benign slow growing tumors of fat cells, preferred located in the subcutaneous fatty tissue that can occur in various forms and characteristics. They can build mucus, chalk and/or become ossified. Additionally, increased built of connective tissue and capsules can occur together with newly built blood vessels which are all classified as abnormal because the compression on the blood vessels as well as on the nerve cells is algetic. Lipomae occur in various syndromes like for example the Gardner syndrome, the Lanois-Bensaude syndrome, and the Proteus syndrome.

Lipomatosis dolorosa and Cellulite are special forms of hypertrophic proliferation of fatty tissue which is located between the dermal fatty fascia and the underside of the dermis. Due to hormonal influences an enhanced capability to bind water in these fatty cells is observed which themselves initiate pressure and cause subsequently congestions in the lymphatic vessels. Additionally, compression and irritation to the peripheral sensitive nerves is applied so that the patients have an extreme sensitivity to contact. Over the years, irregular disseminated localised fatty nodes can built under the thinning dermis which are painful and show an unaesthetic character.

Under the term regression it is in particular understood that the lipolysis of the fatty tissue and the degeneration of the prolific fatty tissue is taking place.

The preparation of an aqueous system of the present invention can for instance be such that at least one surfactant and at least one water dispersible biopolymer are dispersed in water in a ratio disclosed above. The preparation can be brought forward by dissolving in non-aqueous solvents, emulsifying, extrusion, homogenisation or ultra sound application. Alternatively a predispersed biopolymer is incubated with at least one surfactant for at least 1 hour. The surfactant can penetrate the polymer and subsequently a homogenous distribution can be achieved.

The application of an aqueous system of the present invention can be by any form of injection, in particular by subcutaneous injection, by intra-artery injection, by intra-muscular injection or by intravenous injection.
A percutaneous application is also possible in various carrier media. There, various aiding techniques like iontophoresis can be applied. The application can for instance be made via hydrostatic pressure. Thus, an even distribution is achieved.

Preferably, each unit of the formulation has a distinct dose of the aqueous system as active ingredient. This dose can reach from about 10 mg to about 3000 mg, preferred from about 100 mg to about 1000 mg, per overall weight of the surfactant.

For the treatment of an adult patient by application of injection solutions day doses of 5 mg to 5000 mg, preferred of 250 mg to 2500 mg per injection per overall weight of the surfactant dependant of the size of the fatty tissue to be treated are administered.
The dose is also dependant to the size of the fat depot and/or the disordered distribution of the fat cells and/or the type of adipose disease. It should be tailored to the needs of the single patient. In the case of small lipomae even amounts of 10 mg to 50 mg can be used.

### Example:

Beads were manufactured of solid alginate and cross-linked by Barium containing 99% water. The total volume of beads within the solution was >90%, beads had a diameter of 150 µm and were suspended in physiological Ringer solution with a maximum bead density whereas the liquid component inside and outside the beads is the same.
2 g of this bead solution were incubated for about 12 h with 0.4 g polyoxyethylene sorbitan monolaurate. During that time the polyoxyethylene sorbitan monolaurate distributes within the inner and outer phase of the beads.

To investigate the release behaviour the bead dispersion (2 g) was diluted in 20 ml of Ringer solution, mixed and the concentration of polyoxyethylene sorbitan monolaurate measured after centrifugation by UV spectroscopy.

**Table 1:**

| Example 1 | Time [min.] | Concentration [mg/ml] |
|---|---|---|
| | 0 | 0,000000 |
| | 5 | 0,010577 |
| | 10 | 0,015865 |
| | 20 | 0,031730 |
| | 60 | 0,047595 |
| | 120 | 0,063460 |

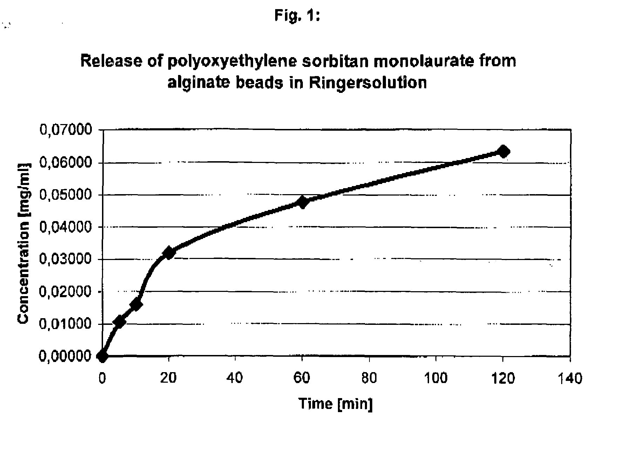

It can be seen from the example and from Fig. 1 that a in a system of the present invention slow release of polyoxyethylene sorbitan monolaurate over a period of two hours can be detected. The example was carried out in Ringer solution which is representative for a physiological environment.

## Claims

1. Aqueous particulate system comprising at least one water dispersible biopolymer, at least one surfactant, and water for the preparation of a formulation for the treatment of adipose diseases and/or conditions.

2. Aqueous system according to claim 1, **characterized in that** the dispersed biopolymer is selected from the group consisting of chitosan, alginates, 3,3-N-(aminoalkyl) chitosan, poly(D,L-lactic-)co-glycoside, lactic acid, glycolic acid, albumin, poly adipic acid anhydride, gellan gum, poly-L-lysine, and polypeptides.

3. Aqueous system according to claim 1 or 2, **characterized in that** the dispersed biopolymer has a particle size between 5 µm and 500 µm, preferably between 50 µm and 200 µm.

4. Aqueous system according to one or more of the preceding claims, **characterized in that** the system additionally comprises an alcohol.

5. Aqueous system according to one or more of the preceding claims, **characterized in that** the surfactant has an HLB value of between 5 and 15.

6. Aqueous system according to one or more of the preceding claims, **characterized in that** the surfactant is selected from the group consisting of 3-sn-phosphatidylcholine, soy (phospholipone 90), reduced soy (phospholipon 90H), 3-(3sn)-phosphatidylgycerol soy (phospholipon G), dimyristoylphosphatidylglycerole, lyso-phophatidylcholine, dipalmitoylphosphatidylglycerole, and/or their physiologically acceptable salts, desoxycholic acid, cholic acid, lithocholic acid, chendodesoxycholic acid, hyodesoxycholic acid, trihydroxycoprostanic acid, ursodesoxycholic acid, taurocholic acid, or glycocholic acid and/or their physiologically acceptable salts, as well as ethers of ethoxylated alcohols and alkyl-alcohols (C6-C16), alkyl-ester with C8-C20 with ethoxylated alcohols, ester of saturated and unsatured acids with C8-C20 with sugars, alkylethersulfates like polyether of caster oil and ethylene oxide (cremephor EL), polyoxyethylene fatty alcohol ether, polysorbic monoester, poloxamer, poloxamine.

7. Aqueous system according to one or more of the preceding claims, **characterized in that** additionally a co-surfactant is comprised.

8. Aqueous system according to claim 7, **characterized in that** the co-surfactant has an HLB value between 9 and 17 and is selected from the group of 3-sn-phosphatidylcholine, soy (phospholipone 90), reduced soy (phospholipon 90H), 3-(3sn)-phosphatidylgycerol soy (phospholipon G), dimyristoylphosphatidylglycerole, lyso-phophatidylcholine, dipalmitoylphosphatidylglycerole, and/or their physiologically acceptable salts, desoxycholic acid, cholic acid, lithocholic acid, chendodesoxycholic acid, hyodesoxycholic acid, trihydroxycoprostanic acid, ursodesoxycholic acid, taurocholic acid, or glycocholic acid and/or their physiologically acceptable salts, as well as ethers of ethoxylated alcohols and alkyl-alcohols (C6-C16), alkyl-ester with C8-C20 with ethoxylated alcohols, ester of saturated and unsatured acids with C8-C20 with sugars, alkylethersulfates like polyether of caster oil and ethylene oxide (cremephor EL), polyoxyethylene fatty alcohol ether, polysorbic monoester, poloxamer, poloxamine.

9. Aqueous system according to one or more of the preceding claims, **characterized in that** additionally at least one lipophilic substance selected from the group consisting of natural oils (e.g. soy bean oil), ester of middle-chain alkylacids with glycols, octyl-dodecanol, silicon oils, paraffins is comprised.

10. Aqueous system according to one or more of the preceding claims, **characterized in that** additionally at least one alcohol, preferably a C2-C8 alsohol, in particular ethanol, propylene glycol, and glycerine, is comprised.

11. Use of an aqueous system according to one of the preceding claims for the preparation of a formulation for the treatment of an adipose disease and/or condition.
